# FASCICULE DE BREVET EUROPEEN

(11) **EP 2 348 278 B1**
(45) Date de publication et mention de la délivrance du brevet: **20.02.2013**
(21) Numéro de dépôt: 10195588.8
(22) Date de dépôt: 17.12.2010
(51) Int. Cl.: G01B 11/06, A61F 5/01, A61F 5/058

(54) **Procédé et appareillage de mesure de l'épaisseur d'une orthèse de contention veineuse élastique à l'état tendu dans des conditions de porté.**
Messverfahren und -apparate zur Dickenmessung einer elastischen Venenkompressionsorthese in gespanntem Zustand unter Tragekonditionen
Method and apparatus for measuring the thickness of an elastic vein compression orthosis in its taut state under wearing conditions

(30) Priorité: 19.01.2010 FR 1050314
(43) Date de publication de la demande: 27.07.2011
(73) Titulaire: Innothera Topic International, 94110 Arcueil (FR)
(72) Inventeur: Cros, François, 94200 Ivry/Seine (FR); Lescaille, Aude, 69009 Lyon (FR); Counord, Jean-Louis, 92500 Rueil-Malmaison (FR)
(74) Mandataire: Dupuis-Latour, Dominique

(56) Documents cités:
- WO-A1-2007/050747
- GB-A- 2 403 006

## Description

L'invention concerne les orthèses de compression veineuse élastique (CVE), qui sont indiquées dans les diverses manifestations cliniques d'insuffisance veineuse des membres inférieurs.

Ces orthèses, anciennement connues sous la dénomination de "bas de contention" ou "chaussettes de contention", sont des dispositifs médicaux textiles produisant un effet thérapeutique par compression/contention des membres inférieurs, par opposition aux "bas de maintien" (ou encore "bas de soutien" ou "bas anti-fatigue") et aux "bas mode" ou "chaussettes mode", qui ne sont pas des dispositifs médicaux à visée thérapeutique. Les orthèses de CVE sont conçues pour produire un effet thérapeutique par compression du membre inférieur sur une étendue plus ou moins grande, avec un profil dégressif vers le haut à partir de la cheville. Selon le type d'orthèse, la pression mesurée à la cheville peut varier de 10 à plus de 36 mmHg (soit 13 à 48 hPa, le mmHg étant toutefois d'usage courant comme unité de mesure de pression dans le domaine de la phlébologie et de la compression médicale).

L'invention concerne plus particulièrement un procédé et un appareillage pour la mesure de l'épaisseur d'un tel article.

Il existe certes une norme NF EN ISO 5084 (novembre 1996) "Détermination de l'épaisseur des textiles et produits textiles*",* qui est une norme générale, non spécifique aux articles de CVE.

Cette norme prévoit la mesure du produit textile à l'état libre, c'est-à-dire à l'état détendu dans le cas d'un article élastique.

Or l'évaluation de l'épaisseur de l'orthèse à l'état tendu (c'est-à-dire dans des conditions de porter) serait utile aux chercheurs et fabricants d'orthèses de CVE, notamment pour la mise au point de nouveaux produits présentant une plus grande finesse au porter. La tendance actuelle va en effet dans le sens d'articles qui, malgré leur fonction thérapeutique, présentent des caractéristiques esthétiques et de confort supérieures, permettant une meilleure acceptation par le patient et conduisant donc à une meilleure observance du traitement de l'insuffisance veineuse.

Lorsqu'une orthèse de CVE est enfilée sur un membre, le textile tendu exerce une compression résultant de la force de rappel des fibres élastiques qui composent le matériau de l'orthèse. Cette déformation des fibres élastiques va avoir une incidence sur l'épaisseur de l'orthèse, de sorte que l'épaisseur de l'article textile à l'état tendu ne sera pas la même qu'à l'état libre, non tendu.

La difficulté est dans ce cas de disposer d'une méthode de mesure permettant de quantifier l'épaisseur de façon rigoureuse et reproductible, méthode qui puisse être utilisée comme méthode de référence, qui soit sujette à un minimum de biais et qui permette des mesures comparatives pour une très grande variété d'orthèses.

Il y a également lieu de tenir compte de la valeur relativement faible de l'épaisseur à mesurer, typiquement de l'ordre de 250-550 µm, le plus souvent dans la plage 300-500 µm.

L'état de la technique dans le domaine médical peut être illustré par le WO 2007/050747 A1, qui décrit une méthode de mesure de l'épaisseur du revêtement d'une endoprothèse par un procédé particulier basé sur l'utilisation d'un microscope à balayage. Dans un tout autre domaine (la technologie des forages), le GB 2 403 006 A décrit la manière de mesurer par voie optique l'espacement entre les spires d'un fil enroulé et le diamètre de ce dernier.

Pour résoudre les difficultés exposées plus haut, l'invention propose un procédé de mesure comprenant les étapes suivantes : mise en place d'une jambe-modèle formant référentiel métrologique, apte à recevoir par enfilage l'orthèse, et d'un fil-étalon, disposé au voisinage de la jambe-modèle de manière à s'étendre, à distance de celle-ci, dans un plan-objet contenant un profil longitudinal de cette jambe-modèle ; formation d'une image du plan-objet contenant le profil de la jambe-modèle ainsi que le fil-étalon; détermination, par analyse de l'image, d'une première distance séparant un point de référence de l'image du fil-étalon, et un point en vis-à-vis de l'image du profil de la jambe-modèle ; enfilage de l'orthèse sur la jambe-modèle ; détermination, par analyse de l'image, d'une seconde distance séparant le point de référence de l'image du fil-étalon, et un point en vis-à-vis de l'image du profil de la jambe-modèle pourvue de l'orthèse enfilée dessus ; et calcul de la différence entre la première et la seconde distance, cette différence étant représentative de l'épaisseur de l'orthèse à l'état tendu dans des conditions de porter.

Selon diverses mises en oeuvre subsidiaires préférentielles:
- le plan-objet est un plan axial de la jambe-modèle ;
- le fil-étalon est un fil rectiligne, et les première et seconde distances sont des distances considérées suivant une direction orthogonale à la direction dans laquelle s'étend le fil-étalon ;
- les distances sont exprimées en pixels d'image, et il est en outre prévu une étape de calcul d'un facteur d'échelle entre, d'une part, un nombre de pixels sur l'image et, d'autre part, une dimension absolue correspondante dans le plan-objet, de manière à délivrer une valeur d'épaisseur de l'orthèse exprimée en unités métriques absolues ;
- l'étape de calcul du facteur d'échelle comprend la détermination le nombre de pixels de l'image de l'image du fil-étalon en direction transversale, et le rapport de ce nombre à un diamètre donné, connu, de ce fil-étalon.

L'invention vise également un appareillage de mesure de l'épaisseur d'une orthèse de CVE à l'état tendu dans des conditions de porter, comprenant les moyens pour la mise en oeuvre du procédé exposé ci-dessus.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

Les Figures 1 et 2 sont des vues, respectivement en plan et en élévation, de l'appareillage selon l'invention de mesure de l'épaisseur d'une orthèse de CVE.

Les Figures 3a et 3b sont des vues agrandies, correspondant au repère III de la Figure 2, permettant d'expliciter la manière dont est évaluée l'épaisseur de l'orthèse par des mesures prises respectivement avant et après enfilage de l'orthèse sur la jambe-modèle.

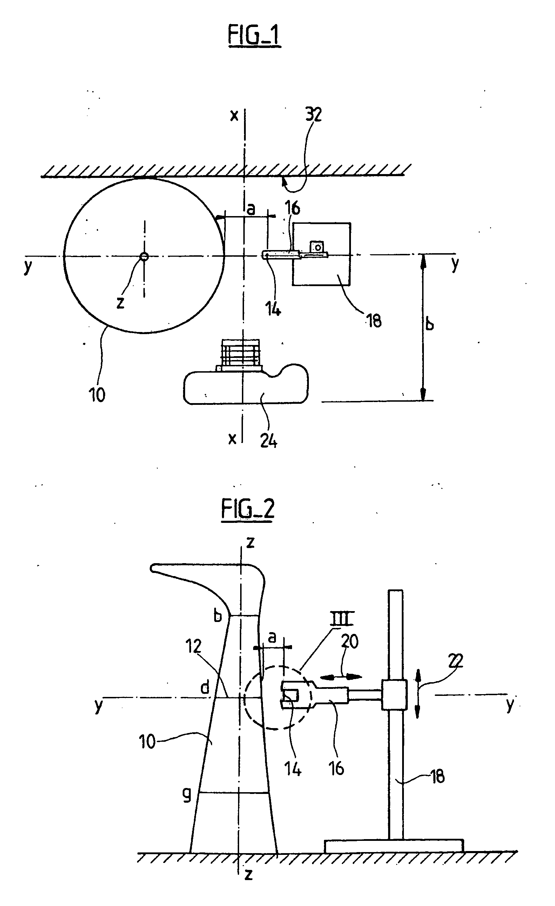

On a représenté sur les Figures 1 et 2 l'appareillage pour la mise en oeuvre du procédé selon l'invention.

Celui-ci comprend une forme sur laquelle peut être enfilée l'orthèse, par exemple une jambe-modèle 10 utilisée comme gabarit métrologique telle que la jambe-modèle de la norme française NF G 30-102b, Annexe B, ou la jambe-modèle type Hohenstein selon le référentiel allemand RAL-GZ 387.

Cette jambe est pourvue de marquages tels que 12 à diverses altitudes normalisées notées conventionnellement b, *b1,* c, *d,* e, f et g. On va par exemple chercher à mesurer l'épaisseur aux altitudes b, d et g, soit :
b : au périmètre minimal de la cheville, à une altitude z = 12 cm par rapport à la plante du pied,
d: au-dessous du genou, à z = 39 cm,
g : en haut de cuisse (pour un produit de type bas-cuisse), à z = 72 cm.

L'appareillage comprend un fil-étalon rectiligne 14, monté sur un support 16 en forme de fourche. Le support 16 est lui-même monté sur une potence 18, avec possibilité d'ajustement en direction horizontale y-y (flèche 20) et en direction verticale z-z (flèche 22).

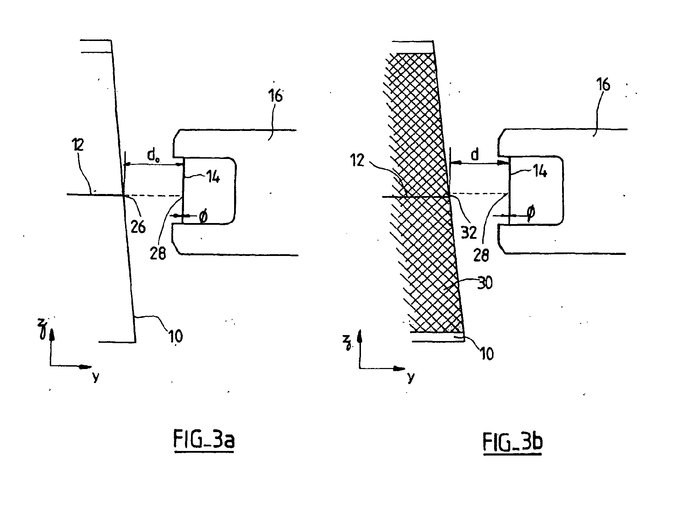

Le fil-étalon 14, visible également sur les Figures 3a et 3b, s'étend verticalement, parallèlement à l'axe principal z-z de la jambe-modèle 10. En outre, comme illustré Figure 1, le fil 14 est situé dans un plan axial *y-y, z-z* de la jambe-modèle 10. Le fil 14 est disposé à une distance a d'environ 2 cm de la surface extérieure de la jambe-modèle. On verra plus loin que cette distance a, par exemple environ 2 cm, n'a pas besoin d'être ajustée avec précision ; il suffit qu'elle soit maintenue inchangée tout au long du processus de mesure.

L'appareillage comprend également un appareil photo numérique 24, fixé sur un statif et orienté suivant une direction horizontale x-x perpendiculaire au plan axial *y-y,* z-z dans lequel s'étend le fil-étalon 14. L'appareil photo numérique 24 est disposé à une distance b d'environ 10 cm du fil-étalon 14. Ici encore, il n'est pas nécessaire que cette distance b soit définie de façon précise ; il suffit qu'elle soit maintenue constante pendant tout le processus de mesure.

A cet égard, il est possible de prévoir un banc de mesure où la potence 18 et le support de l'appareil photo 24 sont solidarisés entre eux.

Un fond 32, disposé en arrière du fil 14 de la jambe-modèle 10, permet d'améliorer le contraste de l'image du fil et de la jambe-modèle qui sera formée par l'appareil photo 24.

Les Figures 3a et 3b illustrent le détail repéré III sur la Figure 2, correspondant à l'image formée par l'appareil photo 24 lorsque celui-ci est mis au point sur le fil-étalon 14, c'est-à-dire lorsque le plan-objet de l'image est le plan *y-y, z-z.*

La Figure 3a illustre l'image obtenue avec la jambe-modèle 10 nue, tandis que la Figure 3b correspond à l'image obtenue, dans les mêmes conditions, avec l'orthèse enfilée sur la jambe-modèle. Entre les deux images, la position des différents éléments (jambe-modèle 10, fil-étalon 14 et son support 16) n'a pas été modifiée, non plus que la position et les réglages de l'appareil photo, la seule différence résidant dans la présence de l'orthèse enfilée sur la jambe-modèle.

Un premier cliché est réalisé avec la jambe-modèle nue, puis un second avec l'orthèse en place. Il convient de noter que la mise en place de l'orthèse 30 doit être réalisée conformément aux prescriptions requises pour ce type d'article. En effet, à la différence d'une chaussette ou d'un bas traditionnel, une orthèse de CVE doit être enfilée avec mise en place progressive et massage au fur et à mesure de l'enfilage, de manière à assurer une distension uniforme des fibres élastiques. En outre, l'enfilage se fera de manière à faire coïncider les repères 12 gravés sur la jambe-modèle 10 avec des marquages préalablement formés sur l'orthèse 30 aux différentes altitudes normalisées (b, d, g, ...). Bien entendu, l'enfilage doit se faire en veillant à ne déplacer aucun élément de l'appareillage de mesure.

Les clichés numériques ainsi obtenus sans orthèse et avec l'orthèse, correspondant respectivement aux Figures 3a et 3b, sont ensuite analysés par un logiciel d'analyse et de traitement d'images numériques, par exemple le logiciel *ImageJ,* qui est un logiciel du domaine public pourvu de diverses fonctions permettant d'exécuter les opérations que l'on va exposer ci-dessous.

La première opération consiste à mesurer sur l'image numérique le diamètre du fil-étalon 14, considéré en direction transversale (y-y), ce diamètre étant exprimé en nombre de pixels sur l'image. Cette opération est effectuée en zoomant sur la partie centrale du fil-étalon (cette partie centrale pourra par exemple être repérée par un marquage de couleur sur le fil), puis en mesurant le nombre de pixels entre chaque bord visible du fil. On connaît d'autre part le diamètre exact du fil-étalon, qui a été mesuré auparavant de façon très précise par des techniques métrologiques conventionnelles (projecteur de profil ou palmer). À partir de cette valeur (par exemple une valeur typique 0 = 0,23 mm), il est possible de calculer un facteur d'échelle permettant de convertir les dimensions mesurées sur l'image, exprimées en nombres de pixels, en unités métriques absolues, par exemple en millimètres, et ceci quelle que soit la précision du positionnement du fil-étalon 14 par rapport à la jambe-modèle 10 (écartement repéré a sur la Figure 1) et de l'appareil photo numérique 26 par rapport au fil-étalon 14 (écartement repéré b sur la Figure 1).

La mesure effectuée entre les deux points 26 et 28 en vis-à-vis de la jambe-modèle 10 sans orthèse et du fil-étalon 14, respectivement, donne une dimension de référence *d₀* (Figure 3a). La mesure effectuée dans les mêmes conditions après enfilage de l'orthèse 30 sur la jambe-modèle 10 donne entre les points 28 et 32 une dimension *d*,.

L'épaisseur de l'orthèse à l'altitude repérée par le marquage 12 est donnée par e = *d₀ - d.*

Ces différents calculs de mise à l'échelle et de mesure d'épaisseur peuvent être opérés de façon automatique à l'aide d'une feuille de calcul d'un tableur, où seront simplement introduites les mesures relevées, exprimées en nombre de pixels.

Il est souhaitable, pour un même produit de CVE, d'effectuer une pluralité de mesure distinctes, et de ne conserver comme valeur finale que la moyenne des différentes épaisseurs mesurées.

## Revendications

1. Un procédé de mesure de l'épaisseur d'une orthèse de contention veineuse élastique à l'état tendu dans des conditions de porter, **caractérisé en ce qu'**il comprend les étapes suivantes :
- mise en place d'une jambe-modèle (10) formant référentiel métrologique, apte à recevoir par enfilage ladite orthèse, et d'un fil-étalon (14), disposé au voisinage de la jambe-modèle de manière à s'étendre, à distance de celle-ci, dans un plan-objet (yOz) contenant un profil longitudinal de cette jambe-modèle ;
- formation d'une image dudit plan-objet contenant le profil de la jambe-modèle ainsi que le fil-étalon ;
- détermination, par analyse de ladite image, d'une première distance (*d₀*) séparant un point de référence (28) de l'image du fil-étalon, et un point en vis-à-vis (26) de l'image du profil de la jambe-modèle ;
- enfilage de l'orthèse (30) sur la jambe-modèle ;
- détermination, par analyse de ladite image, d'une seconde distance (*d*) séparant ledit point de référence (28) de l'image du fil-étalon, et un point en vis-à-vis (32) de l'image du profil de la jambe-modèle pourvue de ladite orthèse (30) enfilée dessus ; et
- calcul de la différence entre la première et la seconde distance, cette différence étant représentative de ladite épaisseur de l'orthèse à l'état tendu dans des conditions de porter.

2. Le procédé de la revendication 1, dans lequel ledit plan-objet (*yOz*) est un plan axial de la jambe-modèle.

3. Le procédé de la revendication 1, dans lequel le fil-étalon (14) est un fil rectiligne, et lesdites première et seconde distances (*d₀*, *d*) sont des distances considérées suivant une direction (*yy*) orthogonale à la direction (*zz*) dans laquelle s'étend le fil-étalon.

4. Le procédé de la revendication 1, dans lequel :
- lesdites distances sont exprimées en pixels d'image, et
- il est en outre prévu une étape de calcul d'un facteur d'échelle entre, d'une part, un nombre de pixels sur ladite image et, d'autre part, une dimension absolue correspondante dans le plan-objet,
de manière à délivrer une valeur d'épaisseur de l'orthèse exprimée en unités métriques absolues.

5. Le procédé de la revendication 4, dans lequel l'étape de calcul du facteur d'échelle comprend la détermination le nombre de pixels de l'image de l'image du fil-étalon (14) en direction transversale (*yy*), et le rapport de ce nombre à un diamètre donné (Ø), connu, de ce fil-étalon.

6. Un appareillage de mesure de l'épaisseur d'une orthèse de contention veineuse élastique à l'état tendu dans des conditions de porter, **caractérisé en ce qu'**il comprend :
- une jambe-modèle (10) formant référentiel métrologique, apte à recevoir par enfilage ladite orthèse ;
- un fil-étalon (14), disposé au voisinage de la jambe-modèle de manière à s'étendre, à distance de celle-ci, dans un plan-objet (*yOz*) contenant un profil longitudinal de cette jambe-modèle ;
- un appareil d'imagerie (24), apte à former une image dudit plan-objet contenant le profil de la jambe-modèle ainsi que le fil-étalon ; et
- des moyens métrologiques, aptes à déterminer par analyse de ladite image du plan objet une différence entre :
• une première distance (*d₀*) séparant un point de référence (28) de l'image du fil-étalon, et un point en vis-à-vis (26) de l'image du profil de la jambe-modèle, en l'absence d'orthèse enfilée sur celle-ci, et
• une seconde distance (*d*) séparant ledit point de référence (28) de l'image du fil-étalon, et un point en vis-à-vis (32) de l'image du profil de la jambe-modèle pourvue de ladite orthèse (30) enfilée dessus,
cette différence étant représentative de ladite épaisseur de l'orthèse à l'état tendu dans des conditions de porter.

7. L'appareillage de la revendication 6, dans lequel ledit plan-objet (*yOz*) est un plan axial de la jambe-modèle.

8. L'appareillage de la revendication 6, dans lequel le fil-étalon (14) est un fil rectiligne, et lesdites première et seconde distances (*d₀*, *d*) sont des distances considérées suivant une direction (*yy*) orthogonale à la direction (*zz*) dans laquelle s'étend le fil-étalon.

9. L'appareillage de la revendication 6, dans lequel :
- lesdites distances sont exprimées en pixels d'image, et
- les moyens métrologiques comprennent des moyens de calcul d'un facteur d'échelle entre, d'une part, un nombre de pixels sur ladite image et, d'autre part, une dimension absolue correspondante dans le plan-objet,
de manière à délivrer une valeur d'épaisseur de l'orthèse exprimée en unités métriques absolues.

10. L'appareillage de la revendication 9, dans lequel les moyens de calcul du facteur d'échelle sont des moyens aptes à déterminer le nombre de pixels de l'image du fil-étalon (14) en direction transversale (*yy*), et à rapporter ce nombre à un diamètre donné (0), connu, de ce fil-étalon.

## Claims

1. Method for measuring the thickness of an elastic vein compression orthosis in its taut state under wearing conditions, **characterized in that** it comprises the following steps:
- setting in place a leg model (10), which forms a metrological reference system and onto which said orthosis can be slipped, and a calibration wire (14), which is arranged in proximity to the leg model in such a way as to extend, at a distance therefrom, in an object plane (yOz) containing a longitudinal profile of this leg model;
- forming an image of said object plane containing the profile of the leg model and also the calibration wire;
- determining, by analysis of said image, a first distance (*d₀*) that separates a reference point (28) of the image of the calibration wire from an opposite point (26) of the image of the profile of the leg model;
- slipping the orthosis (30) onto the leg model;
- determining, by analysis of said image, a second distance (*d*) that separates said reference point (28) of the image of the calibration wire from an opposite point (32) of the image of the profile of the leg model onto which said orthosis (30) has been slipped; and
- calculating the difference between the first distance and second distance, this difference being representative of said thickness of the orthosis in its taut state under wearing conditions.

2. Method of Claim 1, in which said object plane (*yOz*) is an axial plane of the leg model.

3. Method of Claim 1, in which the calibration wire (14) is a rectilinear wire, and said first and second distances (*d₀*, *d*) are distances considered in a direction (*yy*) orthogonal to the direction (*zz*) in which the calibration wire extends.

4. Method of Claim 1, in which:
- said distances are expressed in image pixels, and
- a step is further provided which involves calculating a scaling factor between, on the one hand, a number of pixels on said image and, on the other hand, a corresponding absolute dimension in the object plane,
in such a way as to deliver a value of the thickness of the orthosis expressed in absolute metric units.

5. Method of Claim 4, in which the step of calculating the scaling factor comprises determining the number of pixels of the image of the calibration wire (14) in the transverse direction (*yy*) and the ratio of this number to a known given diameter (0) of this calibration wire.

6. Apparatus for measuring the thickness of an elastic vein compression orthosis in its taut state under wearing conditions, **characterized in that** it comprises:
- a leg model (10), which forms a metrological reference system and onto which said orthosis can be slipped;
- a calibration wire (14), which is arranged in proximity to the leg model in such a way as to extend, at a distance therefrom, in an object plane (*yOz*) containing a longitudinal profile of this leg model;
- an imaging appliance (24), which is able to form an image of said object plane containing the profile of the leg model and also the calibration wire; and
- metrological means, which are able to determine, by analysis of said image of the object plane, a difference between:
• a first distance (*do*) that separates a reference point (28) of the image of the calibration wire from an opposite point (26) of the image of the profile of the leg model, without an orthosis slipped onto the latter, and
• a second distance (*d*) that separates said reference point (28) of the image of the calibration wire from an opposite point (32) of the image of the profile of the leg model onto which said orthosis (30) has been slipped,
this difference being representative of said thickness of the orthosis in its taut state under wearing conditions.

7. Apparatus of Claim 6, in which said object plane (*yOz*) is an axial plane of the leg model.

8. Apparatus of Claim 6, in which the calibration wire (14) is a rectilinear wire, and said first distance and second distance (*d₀*, *d*) are distances considered in a direction (*yy*) orthogonal to the direction (*zz*) in which the calibration wire extends.

9. Apparatus of Claim 6, in which:
- said distances are expressed in image pixels, and
- the metrological means comprise means for calculating a scaling factor between, on the one hand, a number of pixels on said image and, on the other hand, a corresponding absolute dimension in the object plane,
in such a way as to deliver a value of the thickness of the orthosis expressed in absolute metric units.

10. Apparatus of Claim 9, in which the means for calculating the scaling factor are means that are able to determine the number of pixels of the image of the calibration wire (14) in the transverse direction (*yy*) and to establish a relationship between this number and a known given diameter (0) of this calibration wire.

## Patentansprüche

1. Verfahren zur Messung der Dicke einer elastischen Venenkompressionsorthese im gespannten Zustand unter Tragbedingungen, **dadurch gekennzeichnet, dass** es die folgenden Schritte enthält:
- Anordnung eines ein messtechnisches Bezugssystem bildenden Modellbeins (10), das die Orthese durch Aufstreifen aufnehmen kann, und eines Eichdrahts (14), der in der Nähe des Modellbeins angeordnet ist, um sich in Abstand zu diesem in einer Objektebene (yOz) zu erstrecken, die ein Längsprofil dieses Modellbeins enthält;
- Formen eines Bilds der Objektebene, das das Profil des Modellbeins sowie den Eichdraht enthält;
- Bestimmung, durch Analyse des Bilds, eines ersten Abstands (d₀), der einen Bezugspunkt (28) des Bilds des Eichdrahts und einen gegenüberliegenden Punkt (26) des Bilds des Profils des Modellbeins trennt;
- Aufstreifen der Orthese (30) auf das Modellbein;
- Bestimmung, durch Analyse des Bilds, eines zweiten Abstands (d), der den Bezugspunkt (28) des Bilds des Eichdrahts und einen gegenüberliegenden Punkt (32) des Bilds des Profils des Modellbeins trennt, das mit der auf es aufgestreiften Orthese (30) versehen ist; und
- Berechnung der Differenz zwischen dem ersten und dem zweiten Abstand, wobei diese Differenz für die Dicke der Orthese im gespannten Zustand unter Tragebedingungen repräsentativ ist.

2. Verfahren nach Anspruch 1, bei dem die Objektebene (yOz) eine axiale Ebene des Modellbeins ist.

3. Verfahren nach Anspruch 1, bei dem der Eichdraht (14) ein geradliniger Draht ist, und die ersten und zweiten Abstände (d₀, d) Abstände sind, die gemäß einer Richtung (yy) orthogonal zu der Richtung (zz) betrachtet werden, in der sich der Eichdraht erstreckt.

4. Verfahren nach Anspruch 1, bei dem:
- die Abstände in Bildpixeln ausgedrückt werden, und
- außerdem ein Schritt der Berechnung eines Skalierungsfaktors zwischen einerseits einer Anzahl von Pixeln im Bild und andererseits einem entsprechenden Absolutmaß in der Objektebene vorgesehen ist,
um einen Dickenwert der Orthese ausgedrückt in absoluten metrischen Einheiten zu liefern.

5. Verfahren nach Anspruch 4, bei dem der Schritt der Berechnung des Skalierungsfaktors die Bestimmung der Anzahl von Pixeln des Bilds des Eichdrahts (14) in Querrichtung (yy) und des Verhältnisses dieser Anzahl zu einem bekannten gegebenen Durchmesser (Ø) dieses Eichdrahts ist.

6. Gerät zur Messung der Dicke einer elastischen Venenkompressionsorthese im gespannten Zustand unter Tragebedingungen, **dadurch gekennzeichnet, dass** es enthält:
- ein ein messtechnisches Bezugsystem bildendes Modellbein (10), das die Orthese durch Aufstreifen aufnehmen kann;
- einen Eichdraht (14), der in der Nähe des Modellbeins angeordnet ist, um sich in Abstand zu diesem in einer Objektebene (yOz) zu erstrecken, die ein Längsprofil dieses Modellbeins enthält;
- ein Bilderzeugungsgerät (24), das ein Bild der Objektebene formen kann, das das Profil des Modellbeins sowie den Eichdraht enthält; und
- messtechnische Einrichtungen, die durch Analyse des Bilds der Objektebene eine Differenz bestimmen können zwischen:
· einem ersten Abstand (d₀), der einen Bezugspunkt (28) des Bilds des Eichdrahts und einen gegenüberliegenden Punkt (26) des Bilds des Profils des Modellbeins in Abwesenheit einer auf dieses aufgestreiften Orthese trennt, und
· einem zweiten Abstand (d), der den Bezugspunkt (28) des Bilds des Eichdrahts und einen gegenüberliegenden Punkt (32) des Bilds des Profils des Modellbeins, das mit der auf es aufgestreiften Orthese (30) versehen ist, trennt,
wobei diese Differenz für die Dicke der Orthese im gespannten Zustand unter Tragebedingungen repräsentativ ist.

7. Gerät nach Anspruch 6, bei dem die Objektebene (yOz) eine axiale Ebene des Modellbeins ist.

8. Gerät nach Anspruch 6, bei dem der Eichdraht (14) ein geradliniger Draht ist, und die ersten und zweiten Abstände (d₀, d) Abstände sind, die gemäß einer Richtung (yy) orthogonal zu der Richtung (zz) betrachtet werden, in der sich der Eichdraht erstreckt.

9. Gerät nach Anspruch 6, bei dem:
- die Abstände in Bildpixeln ausgedrückt werden, und
- die messtechnischen Einrichtungen Einrichtungen zur Berechnung eines Skalierungsfaktors zwischen einerseits einer Anzahl von Pixeln im Bild und andererseits einem entsprechenden Absolutmaß in der Objektebene enthalten,
um einen Dickenwert der Orthese ausgedrückt in absoluten metrischen Einheiten zu liefern.

10. Gerät nach Anspruch 9, bei dem die Einrichtungen zur Berechnung des Skalierungsfaktors Einrichtungen sind, die die Anzahl von Pixeln des Bilds des Eichdrahts (14) in Querrichtung (yy) berechnen und diese Anzahl in ein Verhältnis zu einem bekannten gegebenen Durchmesser (Ø) dieses Eichdrahts bringen können.
